# EUROPÄISCHE PATENTANMELDUNG

(11) **EP 0 961 117 A1**
(43) Veröffentlichungstag der Anmeldung: **01.12.1999**
(21) Anmeldenummer: 99104328.2
(22) Anmeldetag: 04.03.1999
(51) Int. Cl.: G01N 33/34

(54) **Verfahren und Messgerät zur quantitativen Erfassung von Inhaltsstoffen eines Stoff/Flüssigkeit-Gemisches**

(30) Priorität: 27.05.1998 DE 19823695
(71) Anmelder: Voith Sulzer Papiertechnik Patent GmbH, 89522 Heidenheim (DE)
(72) Erfinder: Griech, Wolfgang, 89522 Heidenheim (DE); Münch, Rudolf, 89551 Königsbronn (DE); Winter, Franz, 73547 Lorch (DE)

(57) **Zusammenfassung**

Ein Meßgerät (10) zur quantitativen Erfassung von Inhaltsstoffen eines Stoff/Flüssigkeit-Gemisches (12) für die Papier- und/oder Kartonproduktion umfaßt wenigstens eine Strahlungsquelle (18) zur Bestrahlung des Gemisches (12) in mehreren definierten unterschiedlichen Wellenlängenbereichen, wenigstens einen Sensor (20) zur Messung der Intensität einer durch das Gemisch (12) beeinflußten Strahlung und wenigstens eine Meß- und/oder Auswerteelektronik (24). Dabei erfaßt jeder Sensor (20) zu einem bestimmten Zeitpunkt lediglich einen der definierten unterschiedlichen Wellenlängenbereiche der Strahlung.

## Beschreibung

Die Erfindung betrifft ein Verfahren sowie ein Meßgerät zur quantitativen Erfassung von Inhaltsstoffen eines Stoff/Flüssigkeit-Gemisches für die Papier- und/oder Kartonproduktion.

Eine Reihe der bisher üblichen Verfahren und Meßgeräte der eingangs genannten Art ist in "Wochenblatt für Papierfabrikation", Heft 7, 1996, Seiten 272 bis 279, beschrieben. Danach wurden Stoffdichtemessungen in einem Konsistenzbereich von 1,5% und höher bisher insbesondere auf der Basis von Scherkraft-Messungen und der Messung der Dielektrizitätskonstante über die Ausbreitungsgeschwindigkeit von Mikrowellen durchgeführt. Stoffdichtemessungen im Niedrigkonsistenzbereich unter 1,5% erfolgten u.a. auf der Basis der Depolarisationsmessung. Dabei wird polarisiertes Licht durch eine Stoffsuspension geführt, woraufhin der polarisierte und der depolarisierte Anteil im gemessenen Licht miteinander verglichen werden. Es wird insbesondere auch ein Meßgerät beschrieben, das die Depolarisation ausgesendeten Laserlichts, die Dämpfung und Rückstreuung von Laser- und Xenonlicht sowie die Absorption von Xenonlicht für verschiedene Wellenlängen mißt. Dabei werden fünfzehn verschiedene optische Meßwerte auf einmal registriert. Aus diesen Meßwerten werden die Faserfeststoff- und Füllstoffkonsistenzen berechnet. Das erhaltene Depolarisationssignal des durchdringenden Lichts ist repräsentativ für die Gesamtkonsistenz. Dämpfung und Rückstreuung dienen der Bestimmung der Füllstoffkonsistenz und der Gesamtkonsistenz. Dieses bekannte Meßgerät wird insbesondere zur Optimierung der Naßpartie verwendet. Dieses Gerät ist zwar in der Lage, mehrere unterschiedliche Inhaltsstoffe des Gemisches zu erkennen. Es ist jedoch relativ aufwendig und damit entsprechend teuer. Zudem ist Durchlicht Voraussetzung, was einen größeren Platzbedarf mit sich bringt. Der Meßkanal muß im Durchmesser klein sein, was insbesondere dann gilt, wenn die Stoffdichte ansteigt. Bei höheren Stoffdichten muß das Medium verdünnt werden.

Für Messungen im Niedrigkonsistenzbereich ist auch bereits ein Gerät bekannt geworden, das auf der Basis der Spitzenwert-Meßmethode mit Durchlicht arbeitet, wobei mittels eines fokussierten Lichtstrahls Fasern gezählt werden und der Aschegehalt durch Absorption gemessen wird. Dieses bekannte Gerät kann zwar pauschal Asche und Fasern unterscheiden. Weitere Unterscheidungsmöglichkeiten bestehen jedoch nicht. Auch hier wird wieder Durchlicht vorausgesetzt, was sich wieder nachteilig auf den Platzbedarf auswirkt. Auch der Meßkanal muß im Durchmesser wieder relativ klein sein, was insbesondere dann gilt, wenn die Stoffdichte ansteigt. Bei höheren Stoffdichten muß das Medium wieder verdünnt werden.

Ziel der Erfindung ist es, ein verbessertes Verfahren sowie ein verbessertes Meßgerät der eingangs genannten Art zu schaffen, bei denen die zuvor angeführten Nachteile beseitigt sind.

Erfindungsgemäß wird diese Aufgabe hinsichtlich des Verfahrens dadurch gelöst, daß das Gemisch mittels wenigstens einer Strahlungsquelle bestrahlt wird, daß die Bestrahlung in mehreren definierten unterschiedlichen Wellenlängenbereichen erfolgt und daß mittels wenigstens eines Sensors die Intensität einer durch das Gemisch beeinflußten Strahlung gemessen wird, wobei mit jedem Sensor zu einem bestimmten Zeitpunkt lediglich einer der definierten unterschiedlichen Wellenlängenbereiche der Strahlung erfaßt wird. Ein Spektrometer ist damit nicht erforderlich.

Dabei kann vorteilhafterweise mittels wenigstens eines Sensors eine von dem Gemisch reflektierte Strahlung erfaßt werden. Zusätzlich oder alternativ ist es auch möglich, mittels wenigstens eines Sensors eine durch das Gemisch hindurchgetretene Strahlung zu erfassen. Die Beschränkung auf die Erfassung einer von dem Gemisch reflektierten Strahlung bringt den Vorteil eines besonders geringen Platzbedarfes mit sich. Ein jeweiliger Sensor kann beispielsweise an einer Umschließung des insbesondere durch eine Suspension gebildeten Gemisches angebracht werden.

Bei einer zweckmäßigen Ausführungsform des erfindungsgemäßen Verfahrens wird das Gemisch mittels wenigstens einer optischen Strahlungsquelle bestrahlt und die Intensität der durch das Gemisch beeinflußten optischen Strahlung mittels wenigstens eines Photoempfängers gemessen.

Wird das Gemisch mittels mehrerer Strahlungsquellen unterschiedlicher Wellenlängenbereiche bestrahlt, so ist es von Vorteil, wenn das Gemisch zeitlich nacheinander mittels der einzelnen unterschiedlichen Strahlungsquellen und/oder mittels unterschiedlicher Kombinationen von Strahlungsquellen bestrahlt wird.

Bei einer zweckmäßigen praktischen Ausführungsform des erfindungsgemäßen Verfahrens wird ein sämtliche unterschiedlichen Wellenlängenbereiche umfassender breitbandiger Sensor verwendet. Nachdem zu einem jeweiligen Zeitpunkt stets nur einer der unterschiedlichen Wellenlängenbereiche der Strahlung erfaßt wird, ist auch hier ein Spektrometer nicht erforderlich.

Bei einer bevorzugten praktischen Ausführungsform wird wenigstens eine Leuchtdiode als Strahlungsquelle verwendet, was insbesondere angesichts der Langlebigkeit sowie der geringen Kosten solcher Bauelemente von Vorteil ist. Damit wird insbesondere auch ein seit langem bestehendes Vorurteil überwunden, wonach solche Leuchtdioden angeblich nicht geeignet sind.

In einem Wellenlängenbereich von 1300 bis 2400 nm gibt es im Absorptionsspektrum zwar relativ starke Peaks wie beispielsweise bei 1450 nm Wasser-Oberton, 1930 nm Wasser, 2100 nm Cellulosefaser, 2010 nm Clay, ca. 2300 nm Latex und Lignin, 2300 bis 2400 nm Polyethylen und andere Kunststoffe, wofür Leuchtdioden nicht verfügbar sind, so daß bisher Lampen mit weißglühenden Glühfäden eingesetzt wurden. Untersuchungen haben jedoch gezeigt, daß durchaus auch mit mehreren LEDs anderer Wellenlängen auf die Inhaltsstoffe des Gemisches geschlossen werden kann. So ist die relative Meßgenauigkeit, d.h. die Ansprechempfindlichkeit eines jeweiligen Sensors auf sehr geringe Konsistenzschwankungen, sehr hoch. Eine möglicherweise geringfügige Einschränkung der absoluten Meßgenauigkeit ist demzufolge unbeachtlich. Von besonderem Vorteil ist auch die im Vergleich zu Lampen mit Glühfäden wesentlich höhere Lebensdauer der Leuchtdioden. Dies ist insbesondere für die Anwendung am Verdünnungswasser-Stoffauflauf entscheidend, da hier üblicherweise 50 bis 150 Sensoren gleichzeitig eingesetzt werden und es für den Betreiber nicht zumutbar ist, laufend mit Lampen-Ausfällen rechnen zu müssen.

Ein zusätzlicher Rückschluß auf die Absorption bei verschiedenen Wellenlängen ist insbesondere dann möglich, wenn wenigstens zwei in unterschiedlicher Entfernung zu den Strahlungsquellen angeordnete Sensoren verwendet werden.

Grundsätzlich ist es auch möglich, jeder Strahlungsquelle jeweils einen eigenen Sensor oder ein eigenes Sensorpaar zuzuordnen. Hierbei ist der Platzaufwand zwar höher. Von Vorteil ist jedoch, daß die über die Sensoren erhaltenen Meßergebnisse gleichzeitig abgefragt werden können.

Die Strahlungsquellen können beispielsweise auch in einer gemeinsamen Umschließung vorgesehen sein und damit praktisch als eine einzige Strahlungsquelle wie beispielsweise als.Lampe mit mehreren verschiedenen Glühfäden verwirklicht sein, bei der dann insbesondere in Abhängigkeit von elektrischen Eingangssignalen die Art der jeweils ausgesendeten Strahlung verändert werden kann.

Die Strahlungsquellen und Sensoren können durch ein Sichtfenster von dem Gemisch getrennt sein. Alternativ ist jedoch auch beispielsweise eine Ankopplung über Spiegelsysteme oder Lichtleiterkabel aus Glas oder Kunststoff denkbar. Der konstruktive Aufwand ist hierbei zwar etwas größer, es wird jedoch Platz eingespart.

Bei einer bevorzugten praktischen Ausführungsform des erfindungsgemäßen Verfahrens werden drei optische Strahlungsquellen und zwei Sensoren verwendet. Damit stehen sechs Signale zur Verfügung. Die benötigte Hardware läßt sich einfach beispielsweise in einem stoffdichtegeregelten Stoffauflauf integrierten, Sie benötigt wenig Platz an der Maschine und ist sehr preisgünstig herstellbar.

Gemäß einer bevorzugten praktischen Ausführungsform wird eine Infrarot-Leuchtdiode (z.B. 880 nm oder 950 nm), eine Rot-Leuchtdiode (z.B. 635 nm) und eine Blau-Leuchtdiode (z.B. 480 nm, eventuell Gallium-Nitrit-LED mit 430 nm) verwendet.

Entscheidend für das Meßverfahren ist der Abstrahlwinkel der Leuchtdioden bzw. LEDs. Vorteilhafterweise wird wenigstens eine Leuchtdiode mit einem Abstrahlwinkel zwischen etwa 12° und etwa 30° verwendet.

Die Schaltfrequenz der Leuchtdioden sollte im Vergleich mit dem Zeitraster, in dem die Konsistenzwerte benötigt werden, hoch sein. Begrenzt wird sie durch die Grenzfrequenzen der Sensoren und der Leuchtdioden sowie durch die Geschwindigkeit der Auswerteelektronik. In der Praxis sind beispielsweise etwa 1000 Schaltvorgänge pro Sekunde oder mehr denkbar. Damit ist es möglich, beispielsweise alle 0,5 Sekunden einen Konsistenz-Meßwert zu erhalten, der selbst wiederum aus der Auswerting von mehr als 500 Einzelmessungen entstanden ist. Dasselbe Ergebnis kann beispielsweise auch dadurch erreicht werden, daß eine Schaltfrequenz von 50 Hz gewählt wird und nach jedem Umschaltvorgang zehn Einzelmeßwerte genommen werden.

Aus den gewonnenen Meßwerten kann beispielsweise durch einen Vergleich mit Erfahrungswerten auf die wichtigsten Inhaltsstoffe des Gemisches geschlossen werden. Es hat sich gezeigt, daß sich eine allgemeine Konsistenzänderung in allen Meßsignalen etwa gleich auswirkt, während eine Änderung der Stoffart sehr unterschiedliche Auswirkungen auf diese Signale besitzt. Durch Experimente läßt sich prüfen, wie sich z.B. eine Änderung der Aschekonzentration, der Faserkonzentration und/oder der Gesamt-Konzentration in den Meßsignalen niederschlägt. Zusätzliche Informationen beispielsweise über die Homogenität des Gemisches können beispielsweise den statistischen Eigenschaften der Vielzahl von Einzelmessungen entnommen werden.

Die betreffenden Kenntnisse werden zweckmäßigerweise in einen entsprechenden Auswerte-Algorithmus eingebracht. In einer betreffenden Steuer- und/oder Auswerteelektronik wird dieser Algorithmus dann dazu verwendet, aus den Meßsignalen die Menge der verschiedenen Inhaltsstoffe zu bestimmen.

Bei einer bevorzugten praktischen Ausführungsform des erfindungsgemäßen Verfahrens wird gleichzeitig die Intensität einer durch das Gemisch beeinflußten optischen Strahlung gemessen und eine Dielektrizitätsmessung durchgeführt, wobei sowohl die Messung der Strahlungsintensität als auch die Dielektrizitätsmessung zur quantitativen Erfassung der Inhaltsstoffe des Gemisches herangezogen wird. Hierbei kann das Gemisch für die Dielektrizitätsmessung insbesondere einer Mikrowellenstrahlung ausgesetzt werden. Zur Intensitätsmessung wird insbesondere wieder eine optische Strahlung wie insbesondere eine Infrarotstrahlung bzw. sichtbares Licht verwendet.

Bei einer zweckmäßigen praktischen Ausführungsform des erfindungsgemäßen Verfahrens werden mehrere Sensoren unterschiedlicher spektraler Empfindlichkeit verwendet. In diesem Fall wird das Gemisch vorzugsweise mittels lediglich einer Strahlungsquelle bestrahlt.

Es kann beispielsweise wenigstens eine Sensor/Filter-Einheit verwendet werden, deren spektrale Empfindlichkeit und/oder Durchlässigkeit einstellbar ist. Die jeweiligen Einstellungen können beispielsweise in Abhängigkeit von elektrischen Eingangssignallen vorgenommen werden.

Bei einer bevorzugten praktischen Ausführungsform des erfindungsgemäßen Verfahrens wird eine durch das Gemisch beeinflußte optische Strahlung sensorseitig spektral aufgeteilt und anschließend auf ein vorzugsweise wenigstens 16 und insbesondere 265 Sensoren umfassendes Photodiodenarray geleitet. So kann beispielsweise ein monolithisches Array mit 256 Sensoren eingesetzt werden. Bei z.B. 256 Spektrallinien ist der Umfang an erhaltener Information beträchtlich. Damit ist eine wesentlich genauere Bestimmung verschiedener Inhaltsstoffe möglich. Da die entsprechenden Bauelemente auf dem Markt immer billiger angeboten werden, steht einer Verwendung einer höheren Anzahl von Strahlungsquellen wie vorzugsweise Leuchtdioden nichts im Wege.

Das erfindungsgemäße Meßgerät ist gekennzeichnet durch wenigstens eine Strahlungsquelle zur Bestrahlung des Gemisches in mehreren definierten unterschiedlichen Wellenlängenbereichen, wenigstens einen Sensor zur Messung der Intensität einer durch das Gemisch beeinflußten Strahlung und wenigstens eine Meß- und/oder Auswerteelektronik, wobei jeder Sensor zu einem bestimmten Zeitpunkt lediglich einen der definierten unterschiedlichen Wellenlängenbereiche der Strahlung erfaßt.

Vorteilhafte Weiterbildungen des erfindungsgemäßen Meßgeräts sind in den Unteransprüchen angegeben.

Das erfindungsgemäße Verfahren bzw. das erfindungsgemäße Meßgerät ist beispielsweise in den Sektionen eines stoffdichtegeregelten Stoffauflaufs einer Papiermaschine verwendbar. Es kann sowohl im Dünnstoffbereich bei Stoffdichten bis etwa 2% als auch im Dickstoffbereich bei Stoffdichten von etwa 2 bis etwa 6% eingesetzt werden.

Es ist insbesondere auch in Zu- und/oder Abflüssen einer Bütte einer Papiermaschine und/oder eines Papiermaschinenteils verwendbar.

Von Vorteil ist auch eine Verwendung im Bereich des Naßteils einer Papiermaschine. Hierbei können die Stoffdichten beispielsweise in einem Bereich von etwa 1% bis etwa 30% liegen.

Das erfindungsgemäße Meßgerät kann beispielsweise auch für das Siebwasser einer Papiermaschine verwendet werden, wo die Stoffdichten in der Regel deutlich kleiner als 1% sind.

Aus dem jeweiligen Meßergebnis kann beispielsweise auf den Mahlgrad, den Luftgehalt und/oder den Grad der Ausflockung in dem insbesondere durch eine Suspension gebildeten Gemisch geschlossen werden. Dabei kann die Meß- und/oder Auswerteelektronik des erfindungsgemäßen Meßgeräts ein jeweiliges Signal liefern, das für die betreffende Größe repräsentativ ist.

Die Erfindung wird im folgenden anhand eines Ausführungsbeispiels unter Bezugnahme auf die Zeichnung näher erläutert; in dieser zeigen:
- Figur 1: in rein schematischer Darstellung eine erste Ausführungsform eines Meßgeräts zur quantitativen Erfassung von Inhaltsstoffen eines Stoff/Flüssigkeit-Gemisches und
- Figur 2: eine rein schematische Darstellung einer weiteren Ausführungsform des Meßgeräts.

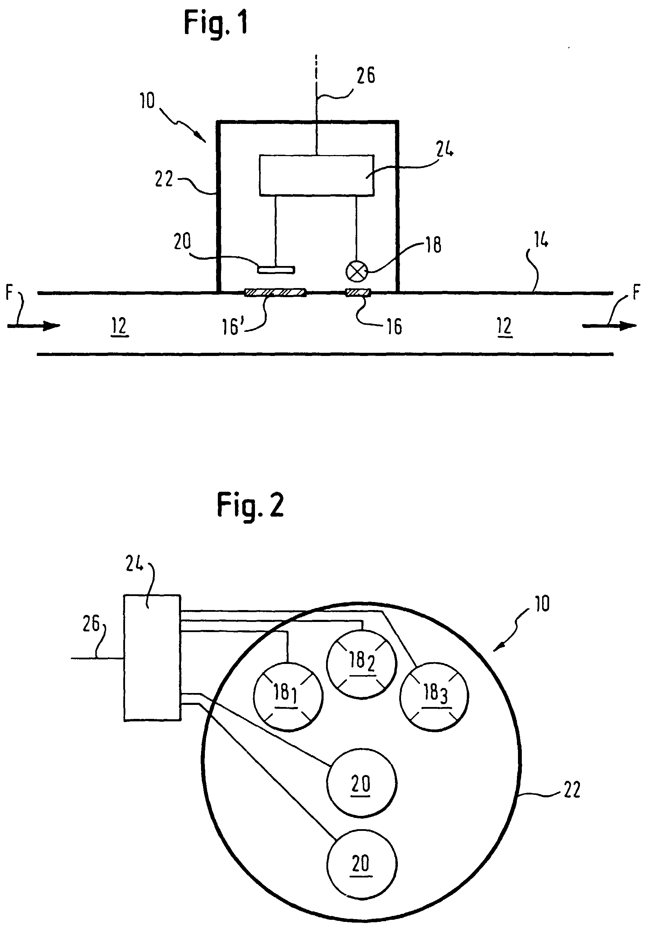

Figur 1 zeigt in rein schematischer Darstellung ein Meßgerät 10, das der quantitativen Erfassung von Inhaltsstoffen eines Stoff/Flüssigkeit-Gemisches 12 für die Papier- und/oder Kartonproduktion dient.

Das Meßgerät 10 ist an einer Umschließung des durch eine Papierfaser-Suspension gebildeten Gemisches 12, im vorliegenden Fall einer Rohrleitung 14, angeordnet. Dabei fließt das Gemisch 12 in Richtung der Pfeile F an im Rohrmantel vorgesehenen Sichtfenstern 16, 16' vorbei, denen wenigstens eine optische Strahlungsquelle 18 wie insbesondere eine Leuchtdiode bzw. wenigstens ein Sensor 20 wie insbesondere ein Photoempfänger gegenüberliegt, die in dem Gehäuse 22 des Meßgeräts 10 angeordnet sind.

In dem Gehäuse 22 des Meßgeräts 10 ist zudem eine Meß- und/oder Auswerteelektronik 24 enthalten, an die die optischen Strahlungsquellen 18 sowie die Sensoren 20 angeschlossen sind. Die Meß- und/oder Auswerteelektronik 24 kann über wenigstens eine Leitung 26 an ein übergeordnetes System, insbesondere ein Prozeßleitsystem und/oder eine Spannungsversorgung, angeschlossen sein.

Die optische Bestrahlung des Gemisches 12 erfolgt in mehreren definierten unterschiedlichen Wellenlängenbereichen. Mittels des wenigstens einen Sensors 18 wird die Intensität der durch das Gemisch 12 beeinflußten Strahlung gemessen. Dabei erfaßt jeder Sensor 18 zu einem bestimmten Zeitpunkt lediglich einen der definierten unterschiedlichen Wellenlängenbereiche der Strahlung.

Beim vorliegenden Ausführungsbeispiel wird lediglich die von dem Gemisch 12 reflektierte Strahlung erfaßt, wodurch der erforderliche Platzbedarf auf ein Minimum herabgesetzt ist.

Figur 2 zeigt in rein schematischer Darstellung eine weitere Ausführungsform des der quantitativen Erfassung von Inhaltsstoffen dienenden Meßgeräts 10.

Wie anhand der Figur 2 zu erkennen ist, enthält das wieder mit einem Gehäuse 22 versehene Meßgerät 10 drei optische Strahlungsquellen 18 und zwei Sensoren bzw. Photoempfänger 20. Sowohl die Strahlungsquellen 18 als auch die Sensoren 20 sind jeweils an die Meß- und/oder Auswerteelektronik 24 angeschlossen, die wieder über wenigstens eine Leitung 26 mit einem übergeordneten System, insbesondere einem Prozeßleitsystem und/oder einer Spannungsversorgung, verbunden sein kann. Im vorliegenden Fall ist die Meß- und/oder Auswerteelektronik 24 außerhalb des Gehäuses 22 vorgesehen.

Die drei optischen Strahlungsquellen werden im vorliegenden Fall durch eine Infrarot-Leuchtdiode 18₁, eine Rot-Leuchtdiode 18₂ und eine Blau-Leuchtdiode 18₃ gebildet. Das Gemisch wird demzufolge durch drei Strahlungsquellen unterschiedlicher Wellenlängenbereiche bestrahlt. Die beiden Sensoren bzw. Photoempfänger 20 sind in unterschiedlicher Entfernung zu den Strahlungsquellen 18 angeordnet.

Die Meß- und/oder Auswerteelektronik 24 steuert den zeitlichen Ablauf der Bestrahlung und Messung. Sie kann z.B. auf dem Gehäuse 22 angeordnet oder, wie im vorliegenden Fall, in einem entfernten Gehäuse untergebracht sein.

Die Meß- und/oder Auswerteelektronik 24 oder ein betreffendes übergeordnetes System kann grundsätzlich wenigstens ein Signal liefern, das beispielsweise für den Mahlgrad, den Luftgehalt und/oder den Grad der Ausflockung in dem insbesondere durch eine Suspension gebildeten Gemisch 12 repräsentativ ist.

### Bezugszeichenliste

- 10: Meßgerät
- 12: Stoff/Flüssigkeit-Gemisch
- 14: Rohrleitung
- 16: Sichtfenster
- 16': Sichtfenster
- 18: optische Strahlungsquelle/Leuchtdiode
- 18₁: Infrarot-Leuchtdiode
- 18₂: Rot-Leuchtdiode
- 18₃: Blau-Leuchtdiode
- 20: Sensor/Photoempfänger
- 22: Gehäuse
- 24: Meß- und/oder Auswerteelektronik
- 26: Leitung
- F: Pfeile

## Patentansprüche

1. Verfahren zur quantitativen Erfassung von Inhaltsstoffen eines Stoff/Flüssigkeit-Gemisches (12) für die Papier- und/oder Kartonproduktion,
dadurch **gekennzeichnet**,
daß das Gemisch (12) mittels wenigstens einer Strahlungsquelle (18) bestrahlt wird, daß die Bestrahlung in mehreren definierten unterschiedlichen Wellenlängenbereichen erfolgt und daß mittels wenigstens eines Sensors (20) die Intensität einer durch das Gemisch (12) beeinflußten Strahlung gemessen wird, wobei mit jedem Sensor (20) zu einem bestimmten Zeitpunkt lediglich einer der definierten unterschiedlichen Wellenlängenbereiche der Strahlung erfaßt wird.

2. Verfahren nach Anspruch 1,
dadurch **gekennzeichnet**,
daß mittels wenigstens eines Sensors (20) eine von dem Gemisch (12) reflektierte Strahlung erfaßt wird.

3. Verfahren nach Anspruch 1 oder 2,
dadurch **gekennzeichnet**,
daß mittels wenigstens eines Sensors (20) eine durch das Gemisch hindurchgetretene Strahlung erfaßt wird.

4. Verfahren nach einem der vorhergehenden Ansprüche,
dadurch **gekennzeichnet**,
daß das Gemisch (12) mittels wenigstens einer optischen Strahlungsquelle (18) bestrahlt und die Intensität der durch das Gemisch beeinflußten optischen Strahlung mittels wenigstens eines Photoempfängers (20) gemessen wird.

5. Verfahren nach einem der vorhergehenden Ansprüche,
dadurch **gekennzeichnet**,
daß das Gemisch mittels mehrerer Strahlungsquellen (18) unterschiedlicher Wellenlängenbereiche bestrahlt wird.

6. Verfahren nach Anspruch 5,
dadurch **gekennzeichnet**,
daß das Gemisch (12) zeitlich nacheinander mittels einzelner Strahlungsquellen (18) unterschiedlicher Wellenlängenbereiche und/oder mittels unterschiedlicher Kombinationen von Strahlungsquellen (18) bestrahlt wird.

7. Verfahren nach wenigstens einem der vorhergehenden Ansprüche,
dadurch **gekennzeichnet**,
daß ein sämtliche unterschiedlichen Wellenlängenbereiche umfassender breitbandiger Sensor (20) verwendet wird.

8. Verfahren nach wenigstens einem der vorhergehenden Ansprüche,
dadurch **gekennzeichnet**,
daß wenigstens eine Leuchtdiode (18) als Strahlungsquelle verwendet wird.

9. Verfahren nach wenigstens einem der vorhergehenden Ansprüche,
dadurch **gekennzeichnet**,
daß wenigstens zwei in unterschiedlicher Entfernung zu den Strahlungsquellen (18) angeordnete Sensoren (20) verwendet werden.

10. Verfahren nach wenigstens einem der vorhergehenden Ansprüche,
dadurch **gekennzeichnet**,
daß jeder Strahlungsquelle (18) jeweils ein eigener Sensor (20) oder ein eigenes Sensorpaar zugeordnet wird.

11. Verfahren nach wenigstens einem der vorhergehenden Ansprüche,
dadurch **gekennzeichnet**,
daß drei optische Strahlungsquellen (18) und zwei Sensoren (20) verwendet werden.

12. Verfahren nach wenigstens einem der vorhergehenden Ansprüche,
dadurch **gekennzeichnet**,
daß wenigstens eine Infrarot-, wenigstens eine Rot- und/oder wenigstens eine Blau-Leuchtdiode verwendet wird.

13. Verfahren nach wenigstens einem der vorhergehenden Ansprüche,
dadurch **gekennzeichnet**,
daß wenigstens eine Leuchtdiode mit einem Abstrahlwinkel zwischen etwa 12° und etwa 30° verwendet wird.

14. Verfahren nach wenigstens einem der vorhergehenden Ansprüche,
dadurch **gekennzeichnet**,
daß gleichzeitig die Intensität einer durch das Gemisch (12) beeinflußten optischen Strahlung gemessen und eine Dielektrizitätsmessung durchgeführt wird und daß sowohl die Messung der Strahlungsintensität als auch die Dielektrizitätsmessung zur quantitativen Erfassung der Inhaltsstoffe des Gemisches (12) herangezogen wird.

15. Verfahren nach Anspruch 14,
dadurch **gekennzeichnet**,
daß das Gemisch (12) für die Dielektrizitätsmessung einer Mikrowellenstrahlung ausgesetzt wird.

16. Verfahren nach wenigstens einem der vorhergehenden Ansprüche,
dadurch **gekennzeichnet**,
daß mehrere Sensoren (20) unterschiedlicher spektraler Empfindlichkeit verwendet werden, wobei das Gemisch (12) vorzugsweise mittels lediglich einer Strahlungsquelle (18) bestrahlt wird.

17. Verfahren nach einem der vorhergehenden Ansprüche,
dadurch **gekennzeichnet**,
daß wenigstens eine Sensor/Filter-Einheit verwendet wird, deren spektrale Empfindlichkeit und/oder Durchlässigkeit einstellbar ist.

18. Verfahren nach einem der vorhergehenden Ansprüche,
dadurch **gekennzeichnet**,
daß eine durch das Gemisch (12) beeinflußte optische Strahlung sensorseitig spektral aufgeteilt und anschließend auf ein wenigstens 16 und insbesondere 265 Sensoren (20) umfassendes Photodiodenarray geleitet wird.

19. Meßgerät (10) zur quantitativen Erfassung von Inhaltsstoffen eines Stoff/Flüssigkeit-Gemisches (12) für die Papier- und/oder Kartonproduktion,
**gekennzeichnet** durch
wenigstens eine Strahlungsquelle (18) zur Bestrahlung des Gemisches (12) in mehreren definierten unterschiedlichen Wellenlängenbereichen, wenigstens einen Sensor (20) zur Messung der Intensität einer durch das Gemisch (12) beeinflußten Strahlung und wenigstens eine Meß- und/oder Auswerteelektronik (24), wobei jeder Sensor (20) zu einem bestimmten Zeitpunkt lediglich einen der definierten unterschiedlichen Wellenlängenbereiche der Strahlung erfaßt.

20. Meßgerät nach Anspruch 19,
dadurch **gekennzeichnet**,
daß wenigstens ein Sensor (20) vorgesehen ist, um eine durch das Gemisch (12) hindurchgetretene Strahlung zu erfassen.

21. Meßgerät nach Anspruch 19 oder 20,
dadurch **gekennzeichnet**,
daß wenigstens ein Sensor (20) vorgesehen ist, um eine von dem Gemisch (12) reflektierte Strahlung zu erfassen.

22. Meßgerät nach einem der vorhergehenden Ansprüche,
dadurch **gekennzeichnet**,
daß wenigstens eine optische Strahlungsquelle (18) vorgesehen ist, um das Gemisch (12) zu bestrahlen, und daß wenigstens ein Photoempfänger (20) vorgesehen ist, um die Intensität der durch das Gemisch (12) beeinflußten optischen Strahlung zu messen.

23. Meßgerät nach einem der vorhergehenden Ansprüche,
dadurch **gekennzeichnet**,
daß mehrere Strahlungsquellen (18) unterschiedlicher Wellenlängenbereiche vorgesehen sind, um das Gemisch (12) zu bestrahlen.

24. Meßgerät nach Anspruch 23,
dadurch **gekennzeichnet**,
daß die Meß- und/oder Auswerteelektronik (24) Mittel umfaßt, um das Gemisch zeitlich nacheinander mittels einzelner Strahlungsquellen unterschiedlicher Wellenlängenbereiche oder/oder mittels unterschiedlicher Kombinationen von Strahlungsquellen zu bestrahlen.

25. Meßgerät nach wenigstens einem der vorhergehenden Ansprüche,
dadurch **gekennzeichnet**,
daß ein sämtliche unterschiedlichen Wellenlängenbereiche umfassender breitbandiger Sensor (20) vorgesehen ist.

26. Meßgerät nach wenigstens einem der vorhergehenden Ansprüche,
dadurch **gekennzeichnet**,
daß wenigstens eine Leuchtdiode (18) als Strahlungsquelle vorgesehen ist.

27. Meßgerät nach wenigstens einem der vorhergehenden Ansprüche,
dadurch **gekennzeichnet**,
daß wenigstens zwei in unterschiedlicher Entfernung zu den Strahlungsquellen (18) angeordnete Sensoren (20) vorgesehen sind.

28. Meßgerät nach wenigstens einem der vorhergehenden Ansprüche,
dadurch **gekennzeichnet**,
daß jeder Strahlungsquelle (18) jeweils ein eigener Sensor (20) oder ein eigenes Sensorpaar zugeordnet ist.

29. Meßgerät nach wenigstens einem der vorhergehenden Ansprüche,
dadurch **gekennzeichnet**,
daß drei optische Strahlungsquellen (18) und zwei Sensoren (20) vorgesehen sind.

30. Meßgerät nach wenigstens einem der vorhergehenden Ansprüche,
dadurch **gekennzeichnet**,
daß wenigstens eine Infrarot-, wenigstens eine Rot- und/oder wenigstens eine Blau-Leuchtdiode (18) vorgesehen ist.

31. Meßgerät nach wenigstens einem der vorhergehenden Ansprüche,
dadurch **gekennzeichnet**,
daß wenigstens eine Leuchtdiode (18) vorgesehen ist, deren Abstrahlwinkel in einem Bereich zwischen etwa 12° und etwa 30° liegt.

32. Meßgerät nach wenigstens einem der vorhergehenden Ansprüche,
dadurch **gekennzeichnet**,
daß die Meß- und/oder Auswerteelektronik (24) Mittel umfaßt, um gleichzeitig die Intensität einer durch das Gemisch (12) beeinflußten optischen Strahlung zu messen und eine Dielektrizitätsmessung durchzuführen, wobei sowohl die Messung der Strahlungsintensität als auch die Dielektrizitätsmessung zur quantitativen Erfassung der Inhaltsstoffe des Gemisches (12) herangezogen wird.

33. Meßgerät nach Anspruch 32,
dadurch **gekennzeichnet**,
daß Mittel vorgesehen sind, um das Gemisch (12) für die Dielektrizitätsmessung einer Mikrowellenstrahlung auszusetzen.

34. Meßgerät nach wenigstens einem der vorhergehenden Ansprüche,
dadurch **gekennzeichnet**,
daß es mehrere Sensoren (20) unterschiedlicher spektraler Empfindlichkeit umfaßt, während vorzugsweise lediglich eine Strahlungsquelle (18) vorgesehen ist, um das Gemisch (12) zu bestrahlen.

35. Meßgerät nach einem der vorhergehenden Ansprüche,
dadurch **gekennzeichnet**,
daß wenigstens eine Sensor/Filter-Einheit vorgesehen ist, deren spektrale Empfindlichkeit und/oder Durchlässigkeit einstellbar ist.

36. Meßgerät nach einem der vorhergehenden Ansprüche,
dadurch **gekennzeichnet**,
daß Mittel vorgesehen sind, um eine durch das Gemisch (12) beeinflußte optische Strahlung sensorseitig spektral aufzuteilen, und daß ein von der aufgeteilten Strahlung beaufschlagbares Photodiodenarray mit wenigstens 16 und insbesondere 265 Sensoren (20) vorgesehen ist.

37. Meßgerät nach einem der vorhergehenden Ansprüche,
dadurch **gekennzeichnet**,
daß die Meß- und/oder Auswerteelektronik (24) wenigstens ein Signal liefert, das für den Mahlgrad, den Luftgehalt und/oder den Grad der Ausflockung in dem insbesondere durch eine Suspension gebildeten Gemisch (12) repräsentativ ist.

38. Verwendung eines Meßgerätes nach einem der vorhergehenden Ansprüche in den Sektionen eines stoffdichtegeregelten Stoffauflaufs einer Papiermaschine.

39. Verwendung eines Meßgerätes nach einem der vorhergehenden Ansprüche in Zu- und/oder Abflüssen einer Bütte einer Papiermaschine und/oder eines Papiermaschinenteils.

40. Verwendung eines Meßgerätes nach einem der vorhergehenden Ansprüche im Bereich des Naßteils einer Papiermaschine.

41. Verwendung eines Meßgerätes nach einem der vorhergehenden Ansprüche für das Siebwasser einer Papiermaschine.
